# EUROPEAN PATENT APPLICATION

(11) **EP 1 598 603 A1**
(43) Date of publication of application: **23.11.2005**
(21) Application number: 02808301.2
(22) Date of filing: 30.12.2002
(51) Int. Cl.: F24F 3/16

(54) **AIR CLEANER**

(71) Applicant: Chiaphua Industries Limited, Tai Po, Hong Kong (CN)
(72) Inventor: WU, Kashing, Kwun Tong, Kowloon, Hong Kong (CN)
(74) Representative: Jenkins, Richard Gavin
(86) International application number: PCT/CN2002/000922
(87) International publication number: WO 2004/059217

(57) **Abstract**

This invention relates to a an air cleaner which includes a housing, a first filter, a photocatalyst reaction unit which can form spiral air flow, a forced convection device and an electrocircuit controller. The forced convection device is provided between the first filter and the photocalatyst reaction unit Because the invention uses a longitudinal air conduct, a air inlet along tangent direction of the air conduction and spiral guide vanes at the inlet, air through a fan and into the air conduct flows spirally in the conduct to increase the time which air contacts with tho pholocalatyst and enable the light of the ultraviolet lamp to radiate on the photocatalyst directly so that the clean efficiency of the photocalatyst is increased effectively.

## Description

### Field of the Invention

The present invention relates to an air cleaning device using photocatalyst, more particularly, to an air cleaning device which has combined effects of sterilization, air filtering and purifying.

### Description of the Related Art

The air cleaning device has been increasingly and widely used as a good varieties of pollution are brought about in the modem society. The air cleaning device typically includes a cooling device, an air purifying device and an exhaust fan etc. In an conventional air purifying device, a corrugated paper material is generally used as a means for separating fume or dust so as to purify the air. Further, active carbon is added in the air cleaning device so that the air is further filtered. That is, the air laden with bacteria can be absorbed by the active carbon contained in the air cleaning device. However, the above conventional air cleaning device is problematic in that the active carbon must be replaced when it is saturated with contaminants removed from the air. More often than not, the consumer may neglect to replace the active carbon. Accordingly, air filtered by the air cleaning device in which the active carbon has not been replaced is not effectively improved in quality.

In recent years, photocatalyst which is mainly composed of TiO₂ has been proposed. The photocatalyst generates a catalyst reaction upon radiation of minute ultra-violet ray so that a sterilization and a deodorization effect on the air laden with bacteria are achieved. In an air cleaning device in which photocatalyst of TiO₂ is applied, a matrix with a complex structure shaped like a honeycomb or a woven web cloth is employed and the matrix or the woven web cloth is impregnated with or sprayed with photocatalyst of TiO₂. However, the above two kinds of conventional air cleaning device have the following disadvantages. For the former one, it is disadvantageous in complicated structure and high cost. For the latter one, it is necessary for the web cloth to be manually cut by the workers. As the specification for the cloth differs from each other in different applications, mass production of the web cloth can not be effected. As a result, no commercialized product of the above air cleaning device is available till now.

Another air cleaning device has been proposed in the Chinese Utility Model Patent No. 00263712.X (Publication No. CN245749Y). The air cleaning device of the above utility model publication is configured as follows. More specifically, the air cleaning device includes a body having an air inlet port and an air outlet port. An active carbon filtering material is disposed adjacent the air inlet port inside the body. The air cleaning device further includes a filtering web. Upon operation of the suction fan, the air is drawn into the air cleaning device through the air inlet port and the air filtered by the active carbon filtering material is sucked and thereafter exhausted outside through the air outlet port. The filtering web is configured to be a structure consisting of plastic web layers which are laminated one by one and are manufactured by injection molding process. Each web layer is provided with a plurality of separation ribs and bore holes. The photocatalyst is provided on the separation ribs. Upon radiation of ultra violet ray, the photocatalyst provided on the separation ribs generates a catalyst reaction so as to decompose air, thus achieving effect of sterilization. As described above, as the filtering web is configured to be a structure consisting of plastic web layers which are laminated one by one in which each web layer is provided with a plurality of separation ribs and bore holes, and which the photocatalyst is provided on the separation ribs, it is possible to achieve mass production of the air cleaning device with above arrangements while reducing the cost of it. Furthermore, the air flowing through the bore holes and gaps therebetween gets into contact with the photocatalyst of TiO₂ so that a sterilization effect and a deodorization effect are achieved. However, it is an important factor for carrying out the catalyst effect that a sufficient amount of ultra violet ray is incident on photocatalyst and the air to be treated must be brought into contact with the photocatalyst. However, as the carrier of the catalyst of the air cleaning device is configured to be a web-layering shape, the light is not evenly incident on the catalyst. Further, the time in which the air contacts with the catalyst is short and the chance for the contact is relatively low. As a result, the purifying efficiency of the photocatalyst is not satisfactory.

### SUMMARY OF THE INVENTION

The present invention has been made to overcome one or more aspects of the above disadvantages in the prior arts. Accordingly, it is an object of the present invention to provide an air cleaning device using photocatalyst in which the chance and time for which the air contacts with the catalyst and the catalytical effect are effectively enhanced and the air purifying efficiency of the catalyst reaction is remarkably increased.

Additional aspects and advantages of the invention will be set forth in part in the description that follows, and in part, will be obvious from the description, or may be learned by the practice of the invention.

The forgoing and other aspects of the present invention are achieved by providing an air cleaning device, comprising: a body; a first filter unit; a photocatalyst reaction unit which generates spiral air current; a forcible convection unit and a circuit control unit which can adjustably control the operation of the forcible convection unit, wherein: the first filter unit is disposed below the body and has a front surface in shape of an opening so as to communicate with the outside and a rear surface in communication with the forcible convection unit, and the forcible convection unit is disposed between the first filter unit and the photocatalyst reaction unit so as to communicate the first filter unit with the photocatalyst reaction unit, characterized in that:
the photocatalyst reaction unit includes an air duct, a photocatalyst coating layer disposed on an interior wall of the air duct, two lamp holders, at least one ultra violet ray tube mounted on the two lamp holders, and a blow guide holder on which a spiral blow guide blade is mounted,
   wherein two ends of the air duct are hermetically connected to left and right side plates of the body respectively,
the air duct is provided at a left side thereof with an air inlet port which is in communication with the air outlet port of the forcible convection unit in a tangential direction thereof;
two ends of each ultra violet ray tube are mounted on the lamp holders and axially disposed inside the air duct;
the blow guide holder is provided on the left side plate and located at a position of the air inlet port of the air duct;
the blow guide holder is provided at a right end thereof with a plurality of vent holes which are formed and arranged in a form of loop; and
a vent opening which is in communication with the vent holes is provided at a side wall of the blow guide holder.

According to the present invention, a photocatalyst reaction unit in which an elongated air duct is combined with an air inlet port disposed in a tangential direction of the air duct and spiral guide blades disposed at the air inlet port thereof is employed. The air drawn into the air duct through the blower flows spirally inside the air duct along the interior wall of the air duct. As a result, the time and chance for which the air contacts with the photocatalyst are greatly increased.

With this construction, ultra violet ray emitted from the ultra violet ray tube can be directly incident onto the photocatalyst without blocking so that the catalyst reaction effect is greatly enhanced. Thus, the purifying efficiency of the air cleaning device is further increased. Particularly, since the specific gravity of the volatile organic chemical (VOC) and bacteria is greater than that of the air, the volatile organic chemical (VOC) and bacteria will be certainly thrown against the interior wall of the air duct when the air rotates around the central axis of the air duct due to the eccentric forces applied thereupon. In this way, the volatile organic chemical (VOC) and bacteria will come more closely to the photocatalyst. Consequently, the photocatalyst causes the volatile organic chemical (VOC) and bacteria to be decomposed into free ion radicals so that the effects of sterilization, deodorization and purification of air can be achieved.

Particularly, it is preferably employed that the air outlet port and the air inlet port are disposed at the same end. When the air rotationally flows from the air inlet port to the distal end of the air duct, the air returns back to air inlet port end along the central portion of the air current (i.e. the position where the ultra violet ray tubes are located) so as to be discharged from the vent opening due to vortex effect of air current (the center of the spiral air current where the pressure is smallest corresponds to the central axis of the air duct i.e. the position adjacent to where the ultra violet ray tubes are located). Thus, the time and chance for performing sterilization by the ultra violet ray tubes are increased.

Moreover, due to generation of the central air current, the air drawn into the air duct is forced toward to the interior wall of the air duct so that the time and chance for which the air contacts with the photocatalyst are increased. As a result, the purifying efficiency of the air cleaning device is further enhanced.

The air cleaning device of the present invention has a simple construction and can be easily effected with good applicability. The air cleaning device according to the present invention can be widely used as a purifying device in rooms inside a house, automobiles or a good variety of appliances such as air conditioners and dishwashers for dust-removing, sterilization, and air-cleaning.

### BRIEF DESCRIPTION OF THE DRAWINGS

The above and other objects and advantages of the invention will become apparent and readily appreciated from the following description of the preferred embodiments, taken in conjunction with the accompanying drawings, in which:
Fig. 1 is an exploded view showing an air cleaning device according to an embodiment of the present invention;
Fig.2 is sectional view of an air cleaning device according to an embodiment of the present invention;
Fig.3 is a schematic view showing the construction of a lamp holder and a ultra violet ray tube of the present invention;
Fig.4 is a schematic view showing the construction of the air duct of the present invention.

### DETAILED DESCRIPTION OF THE PREFERRED EMBODIMENTS

A preferred embodiment of the present invention will be described hereinafter in detail with reference to the attached drawings, wherein the like reference numerals refer to the like elements throughout the specification. The present invention may, however, be embodied in many different forms and should not be construed as being limited to the embodiment set forth herein; rather, this embodiment is provided so that the present disclosure will be thorough and complete, and will fully convey the concept of the invention to those skilled in the art.

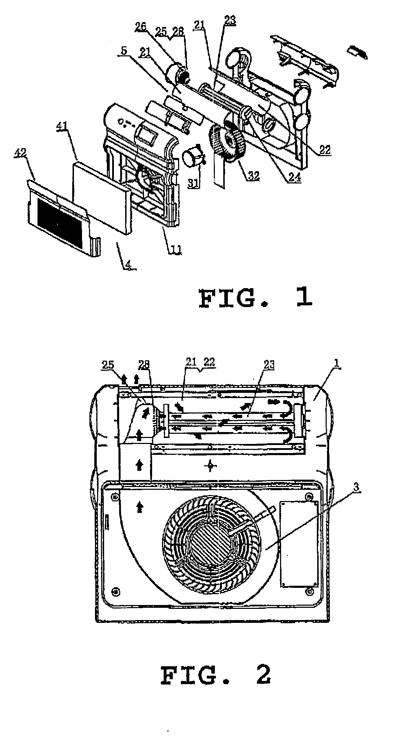

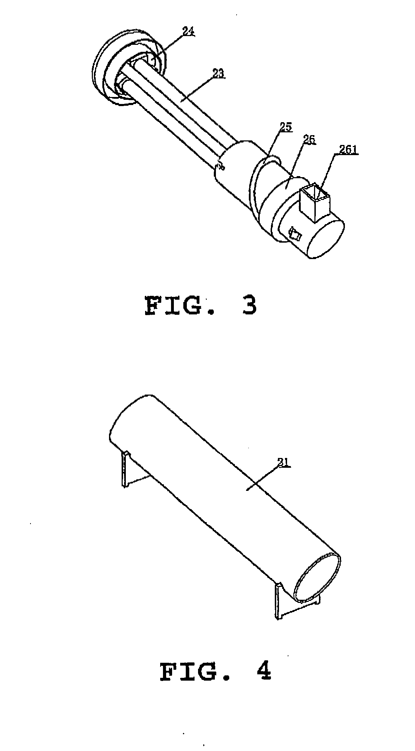

As shown in Figs 1-4, the air cleaning device of the present invention includes a body 1, a first filter unit 4 for filtering the air so as to remove contaminants and dust from the air, a photocatalyst reaction unit which generates spiral air current; a forcible convection unit 3 which forcibly draws air from outside into the first filter unit 4 and sends it into the photocatalyst reaction unit, and a circuit control unit 5 which can adjustably control the operation of the forcible convection unit 3. The first filter unit 4 is disposed below the body 1 having a front surface in shape of an opening so as to communicate with the outside and a rear surface in communication with an inlet port of the forcible convection unit 3. The forcible convection unit 3 is disposed between the first filter unit 4 and the photocatalyst reaction unit so as to communicate the first filter unit 4 with the photocatalyst reaction unit. The air which has been filtered by the first filter unit 4 is delivered into the photocatalyst reaction unit so that the catalyst reaction is carried out therein. The circuit control unit 5 is provided inside the body 1, and a plurality of control buttons and a display unit are provided on a control panel of the body 1. With this construction, the operations of the photocatalyst reaction unit and the forcible convection unit 3 can be adjustably controlled by manipulation of the buttons provided on the control panel of the body 1.

The first filter unit 4 includes a dust blocking web 41 which is provided on a front housing 11 of the body 1 and a movable door 42. The dust blocking web 41 is a filter web made of active carbon or high-efficiency HEPA filtering materials or a combination thereof. The movable door 42 is disposed on the front side of the dust blocking web 41 and provided with an air suction grill. The forcible convection unit 3 is configured to be a blower consisting of a motor 31 which is provided between a front housing and a rear housing of the body and connected to the circuit control unit 5 and a plurality of blades 32 which are mounted on a rotation shaft of the motor 31. An air inlet port of the blower is in communication with the first filter unit 4 and an air outlet port thereof is in communication with an air inlet port of the photocatalyst reaction unit. In order to effectively generate spiral air current, the photocatalyst reaction unit includes an elongated air duct 21, a photocatalyst coating layer 22 disposed on an interior wall of the air duct 21, two lamp holders 24, at least one ultra violet ray tube 23 mounted on the two lamp holders 24 and a blow guide holder 26 on which a spiral blow guide blade 25 is mounted. The air duct 21 is composed of two elongated housings each having a semi-circle section which can be abutted with each other. A recess is provided on the left lower side of each of the two semi-circle shaped housings. Accordingly, the recesses provided on the two semicircle shaped housings respectively can be abutted with each other so as to form an air inlet port.

The interior wall of the air duct 21 can be formed into a smooth surface or an accidented surface with undulations. In an exemplified embodiment of the present invention, the interior wall of the air duct 21 is formed into an accidented surface with undulations. The photocatalyst coating layer 22 is coated onto the accidented surface of the interior wall of the air duct by a spraying or impregnating process. Consequently, the ratio surface area of the photocatalyst coating layer can be effectively increased so that the purifying efficiency can be enhanced. Two ends of the air duct 21 are hermetically connected to left and right side plates of the body 1. The air duct is provided at the left side thereof with an air inlet port which is in communication with the air outlet port of the forcible convection unit in a tangential direction thereof. In this way, since the air inlet port of the air duct is inclined and the air inlet port is disposed in a tangential direction of the air duct 21, the air drawn into the air duct 21 can reliably flow along the interior wall of the air duct 21 so as to enhance the contacting between the air and the photocatalyst only by adjusting the blowing speed of the forcible convection unit 3.

In this case, three ultra violet ray tubes 23 are arranged in form of a Chinese Character " 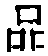 " . Two ends of each ultra violet ray tube 23 are mounted on the lamp holders 24 and are axially disposed inside the air duct 21. With this construction, ultra violet ray emitted from the ultra violet ray tube 23 can be directly incident onto the photocatalyst without blocking so that the catalyst reaction is enhanced into the most desirable state. Thus, the purifying efficiency of the air cleaning device is further increased.

The blow guide holder 26 on which a spiral blow guide blade 25 is mounted is provided on the left side plate of the body 1 and located at a position of the air inlet port of the air duct 21. Due to rotation of the blow guide blade 25, the air flows spirally inside the air duct 21. As a result, the time and the chance for which the air contacts with the photocatalyst are improved, thus enhancing purifying efficiency of the air cleaning device of the present invention.

Further, the blow guide holder 26 is provided with a plurality of vent holes 28 which are formed and arranged in the form of a loop. A vent opening 261 which is in communication with the vent holes is provided a side wall of the blow guide holder 26. With this construction in which the air outlet port and the air inlet port are provided on the same end of the air duct 21, the time and chance for performing sterilization by the ultra violet ray tubes 23 are increased. More specifically, when the air rotationally flows from the air inlet port to the distal end of the air duct 21, the air returns back to air inlet port end along the central portion of the air current (i.e. the position where the ultra violet ray tubes 23 are located) so as to be discharged from the vent opening 261 due to vortex effect of air current (the center of the spiral air current where the pressure is smallest corresponds to the central axis of the air duct 21 .i.e. the position adjacent to where the ultra violet ray tubes 23 are located). Thus, the time and chance for performing sterilization by the ultra violet ray tubes 23 are increased.

Moreover, due to generation of the central air current, the air drawn into the air duct is forced toward to the interior wall of the air duct so that the time and chance for which the air contacts with the photocatalyst are increased. As a result, the purifying efficiency of the air cleaning device is further enhanced.

In addition, one of the two lamp holders 24 is connected to the right side plate of the body 1, and the other one is connected to the blow guide holder 26. With this simple construction, the lamp holders, the air duct, the ultra violet ray tubes and the blow guide blade are integrally formed into a single assembly.

## Claims

1. An air cleaning device, comprising: a body (1); a first filter unit (4); a photocatalyst reaction unit which generates spiral air current; a forcible convection unit (3) and a circuit control unit (5) which can adjustably control the operation of the forcible convection unit (3),wherein: the first filter unit (4) is disposed below the body (1) and has a front surface in shape of an opening so as to communicate with the outside and a rear surface in communication with an inlet port of the forcible convection unit (3), and the forcible convection unit (3) is disposed between the first filter unit (4) and the photocatalyst reaction unit so as to communicate the first filter unit (4) with the photocatalyst reaction unit, **characterized in that**:
the photocatalyst reaction unit includes an air duct (21), a photocatalyst coating layer (22) disposed on an interior wall of the air duct (21), two lamp holders (24), at least one ultra violet ray tube (23) mounted on the two lamp holders (24), and a blow guide holder (26) on which a spiral blow guide blade (25) is mounted,
wherein two ends of the air duct (21) are hermetically connected to left and right side plates of the body (1) respectively,
the air duct is provided at a left side thereof with an air inlet port which is in communication with the air outlet port of the forcible convection unit (3) in a tangential direction thereof;
two ends of each ultra violet ray tube (23) are mounted on the lamp holders (24) and axially disposed inside the air duct (21);
the blow guide holder (26) is provided on the left side plate and located at a position of the air inlet port of the air duct (21);
the blow guide holder (26) is provided at a right end thereof with a plurality of vent holes (28) which are formed and arranged in form of a loop; and
a vent opening (261) which is in communication with the vent holes is provided at a side wall of the blow guide holder (26).

2. The air cleaning device according to claim 1, **characterized in that**:
the air duct (21) is composed of two elongated housings each having a semi-circle section which can be abutted with each other, wherein each of the two semi-circle shaped housings is provided at a lower left side thereof with a recess so that the two recesses of the two housings can be abutted with each other so as to form an air inlet port.

3. The air cleaning device according to claim 1 or 2, **characterized in that**:
the interior wall of the air duct (21) is formed into an accidented surface with undulations, and
the photocatalyst coating layer (22) is coated onto the accidented surface of the interior wall of the air duct by a spraying or impregnating process.

4. The air cleaning device according to claim 1 or 2, **characterized in that**:
the first filter unit (4) includes a dust blocking web (41) and a movable door (42) which are provided on a front housing (11) of the body (1), wherein:
the dust blocking web (41) is a filter web made of active carbon or high-efficiency HEPA filtering materials or a combination thereof; and
the movable door (42) is disposed on the front side of the dust blocking web (41) and provided with an air suction grill.

5. The air cleaning device according to claim 1 or 2, **characterized in that**:
the forcible convection unit (3) is configured to be a blower consisting of a motor (31) which is provided between a front housing and a rear housing of the body (1) and connected to the circuit control unit (5), and a plurality of blades (32) which are mounted on a rotation shaft of the motor (31), wherein:
an air inlet port of the blower is in communication with the first filter unit (4) and an air outlet port thereof is in communication with an air inlet port of the photocatalyst reaction unit.
